**Europäisches Patentamt**

(19) **European Patent Office**

**Office européen des brevets**

(11) Veröffentlichungsnummer: **0 064 547**
**B1**

(12) # EUROPÄISCHE PATENTSCHRIFT

(45) Veröffentlichungstag der Patentschrift:
13.11.85

(51) Int. Cl.⁴: **C 12 P 1/04,** C 12 N 1/20 //
(C12P1/04, C12R1:01)

(21) Anmeldenummer: **81903261.6**

(22) Anmeldetag: **19.11.81**

(86) Internationale Anmeldenummer:
**PCT/EP 81/00183**

(87) Internationale Veröffentlichungsnummer:
**WO 82/01721 (27.05.82 Gazette 82/14)**

(54) **WIRKSTOFFE UND VERFAHREN UND MIKROORGANISMEN ZU DEREN HERSTELLUNG.**

Verbunden mit 81109783.1/0052867 (europäische Anmeldenummer/Veröffentlichungsnummer) durch Entscheidung vom 26.07.84.

(30) Priorität: 19.11.80 DE 3043642
17.11.81 DE 3145621

(43) Veröffentlichungstag der Anmeldung:
17.11.82 Patentblatt 82/46

(45) Bekanntmachung des Hinweises auf die Patenterteilung:
13.11.85 Patentblatt 85/46

(84) Benannte Vertragsstaaten:
DE

(56) Entgegenhaltungen:
EP - A - 0 022 425

T. Korzybski "Antibiotics", Band 1, 1967, Pergamon press, Warszawa PWN, "Antibiotics from myxococcaceae", Seite 159
Chemical Abstracts, Band 75, Nr. 17, 25. Oktober 1971, (Columbus, Ohio, US), Haska, Guno "Extracellular lytic enzymes of Myxococcus virescens. I. Separation of the bacteriolytic enzymes from the bulk of the proteinases", Seite 30, Ref. 105335q

(73) Patentinhaber: **Gesellschaft für Biotechnologische Forschung mbH (GBF), Mascheroder Weg 1, D-3300 Braunschweig-Stöckheim (DE)**

(72) Erfinder: **AUGUSTINIAK, Hermann, In den Lindendöhren 19, D-3340 Wolfenbüttel (DE)**
Erfinder: **GERTH, Klaus, Am Butterbusch 17, D-3300 Braunschweig (DE)**
Erfinder: **HÖFLE, Gerhard, Am Windmühlenberg 2, D-3300 Braunschweig (DE)**
Erfinder: **IRSCHIK, Herbert, Alter Weg 5, D-3340 Wolfenbüttel (DE)**
Erfinder: **KEMMER, Torsten, verstorben, D-3340 Wolfenbüttel (DE)**
Erfinder: **KOHL, Werner, Wolfenbütteler Strasse 46, D-3300 Braunschweig (DE)**
Erfinder: **KUNZE, Brigitte, Wolfenbütteler Strasse 43, D-3300 Braunschweig (DE)**
Erfinder: **REICHENBACH, Hans, Platanenstrasse 35, D-3340 Wolfenbüttel (DE)**
Erfinder: **SANTOSO, Sentot, Admonterring 51, D-6301 Pohlheim 2 (DE)**
Erfinder: **STEINMETZ, Heinrich, Sandgrubenweg 32, D-3300 Braunschweig (DE)**
Erfinder: **TROWITZSCH, Wolfram, Im Dorfe 17a, D-3300 Braunschweig (DE)**

(74) Vertreter: **Boeters, Hans Dietrich, Dr. et al, Boeters, Bauer & Partner Thomas-Wimmer-Ring 14, D-8000 München 22 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Wirkstoffe, die unter Verwendung neuer Mikroorganismen gewonnen werden sowie diese Mikroorganismen. Ferner betrifft die Erfindung ein Verfahren zur Herstellung der Wirkstoffe.

### Herkunft und Hinterlegung der Mikroorganismen

Bei den neuen Mikroorganismen handelt es sich um Stämme des Bakteriums Myxococcus virescens der Klasse Flexibacteriae, der Ordnung Myxobacterales und der Familie Myxococcaceae. Der Stamm MX-v-48 wurde im November 1977 aus einer Probe von Kaninchenmist, der im April 1977 im Osten Teneriffas, Spanien, bei Punta del Hidalgo, gesammelt worden war, isoliert. Hinterlegungen bei der Deutschen Sammlung von Mikroorganismen/Göttingen:

| Bezeichnung | Hinterlegungs-Nr. | Hinterlegungstag |
|---|---|---|
| Mx-v-48 | DSM 1888 | 19. 8. 1980 |
| Mx-v-48-M(utante) 8 | DSM 2177 | 13. 11. 1981 |

### Beschreibung des Stamms Mx-v-48 (DSM 1888)

Die vegetativen Zellen sind zylinderförmige Stäbchen von etwa 4 bis 6 µm Länge und einer Dicke von etwa 0,8 bis 0,9 µm mit sich verjüngenden Enden (»zigarrenförmig«).

Die Myxosporen weisen eine angenäherte Kugelform mit einem Durchmesser von etwa 1,8 bis 2,3 µm auf und erscheinen im Phasenkontrastmikroskop stark lichtbrechend.

Die Zellen bewegen sich gleitend fort, und die Kolonien breiten sich schwarmförmig allmählich auf der Oberfläche einer Kulturplatte aus.

Auf einem peptonhaltigen Kultursubstrat (z. B. cy-Agar) zeigt der Schwarm meist eine kräftige gelbgrüne Färbung.

— cy-Agar:

| | |
|---|---|
| 0,3% | Casitone (Difco) |
| 0,1% | Hefe-Extrakt (Difco) |
| 0,1% | $CaCl_2 \times 2\ H_2O$ |
| 1,5% | Agar |
| pH 7,2 | |

Auf Agar mit Bäckerhefe (z. B. vy/2-Agar) ist der Stamm meist farblos. Hefezellen werden kräftig lysiert.

— vy/2-Agar:

| | |
|---|---|
| 0,5% | Bäckerhefe (bezogen auf Frischgewicht) |
| 0,1% | $CaCl_2 \times 2\ H_2O$ |
| 0,5 mg/l | Cyanocobalamin |
| 1,5% | Agar |
| pH 7,2 | |

Auf vy/2-Agar werden oft weichschleimige, tropfenförmige Fruchtkörper von etwa 200 bis 300 µm Höhe und etwa 200 bis 300 µm Durchmesser gebildet, welche eine gelbgrüne oder auch orange bis rote Färbung aufweisen.

In Flüssigmedien wächst der Organismus in homogener Suspension. Beispielsweise werden die Kulturen in Schüttelkolben (z. B. 50 ml in 250-ml-Erlenmeyerkolben) bei 30°C (mit z. B. 160 U/min) geschüttelt. Als Medium läßt sich beispielsweise cas l.m. verwenden:

| | |
|---|---|
| 1% | Casitone (Difco) |
| 0,1% | $MgSO_4 \times 7\ H_2O$ |
| pH 6,8 | |

Der Stamm Mx-v-48 (DSM 1888) läßt sich mit folgenden Konservierungsmethoden über längere Zeit am Leben erhalten:

1) Myxosporen in Magermilch getrocknet,
2) vegetative Zellen in cas l.m. bei −80° C eingefroren,
3) Fruchtkörper auf Filterpapier getrocknet.

Der Stamm MNx-v-48 (DSM 1888) ist auf die Verwertung von Proteinen und Peptonen eingestellt und baut andere Mikroorganismen (Bakterien und Hefen) ab.

Durch Kultivieren von Myxococcus virescens Mx-v-48 (DSM 1888) in einem Kohlenstoff, Stickstoff und Mineralsalze enthaltenden wäßrigen Nährmedium bei einer Temperatur von etwa 15 bis 40° C ist eine Kulturbrühe erhältlich. Vorzugsweise ist die Kulturbrühe durch Kultivieren von Myxococcus virescens Mx-v-48 (DSM 1888) bei Temperaturen von etwa 25 bis 35° C, insbesondere bei 30° C, erhältlich.

Die Überstände von Flüssigkulturen des Organismus zeigen nach Konzentrieren eine deutliche Aktivität gegenüber gram-negativen Bakterien, insbesondere gegenüber Enterobacteriaceae, jedoch können auch gram-positive Bakterien im Wachstum gehemmt werden.

Die Wirkstoffe (Antibiotika) werden während der exponentiellen Wachstumsphase gebildet. Der optimale Zeitpunkt für den Abbruch der Fermentation kann mittels Messung der optischen Dichte (gemessen bei lambda 623 nm und einer Schichtdicke von 1 cm; o.D.$_{623}$) durch ein Eppendorf-Photometer bestimmt werden. Er ist erreicht, wenn die o.D.$_{623}$ bei Werten von etwa 0,8 bis 1,2 liegt. Die Kultur wächst anschließend noch linear weiter und erreicht bei Vorlage von 0,5% Pepton eine maximale optische Dichte o.D.$_{623}$ nm von etwa 2. Zu diesem Zeitpunkt sind die Wirkstoffe nicht mehr nachweisbar. Extrakte mit antibiotischer Aktivität, ein Wirkstoffgemisch und Wirkstoffe können aus dem Kulturüberstand gewonnen werden. In den Zeilen ist die Aktivität sehr gering.

Im Verlauf einer Stammoptimierung wurde durch mehrfache Mutagenisierungsschritte eine Produktionsmutante Mx-v-48 M8 (DSM 2177) erzeugt.

Diese Mutante ist gegenüber ihren Antibiotika resistent bis 35 µg/ml. Die Generationszeit ist im Vergleich mit dem Wildstamm unverändert, auch in Gegenwarte von 20 µg Antibiotika/ml.

Während der Wildstamm die Antibiotika nur während der logarithmischen Wachstumsphase produziert und die Aktivität in der stationären Wachstumsphase wieder verschwindet, produziert die Mutante erst gegen Ende der log Phase mit Beginn einer auftretenden Substratlimitierung. Die Ausbeute an Antibiotika liegt bei einer typischen Fermentation bei 2 mg/ml.

Durch Abtrennen der Zellen von Myxococcus virescens Mx-v-48 M8 (DSM 2177) von einer erfindungsgemäß erhaltenen Kulturbrühe und Extrahieren der abgetrennten Kulturbrühe mit einem organischen Lösungsmittel, wie beispielsweise Essigsäureethylester, ist eine Extraktphase erhältlich.

Alternativ wird die Extraktphase auch erhalten, wenn Adsorberharz auf Polystyrolbasis (Amberlite XAD-2 oder XAD-4) in die Kulturbrühe eingerührt wird, das Harz abfiltriert, mit Wasser gewaschen und anschließend mit Aceton eluiert wird. Die Extraktphase läßt sich dabei von weiteren Verunreinigungen befreien, wenn vor der Elution mit Aceton Wasser : Methanol = 1 : 1 zur Eluierung verwendet wird. Diese Methode ist auch als Chromatographie an einer Säule durchführbar.

Aus der erfindungsgemäß erhältlichen Extraktphase läßt sich nach deren Entwässerung und Einengung durch

(a) Verteilen zwischen einer polaren und einer unpolaren organischen Phase,
(b) Chromatrographieren der eingeengten polaren Phase beispielsweise über Sephadex L-20 und
(c) Chromatographieren an Reversed-Phase-Kieselgel mit einem polaren organischen Lösungsmittel unter Zusatz von Wasser

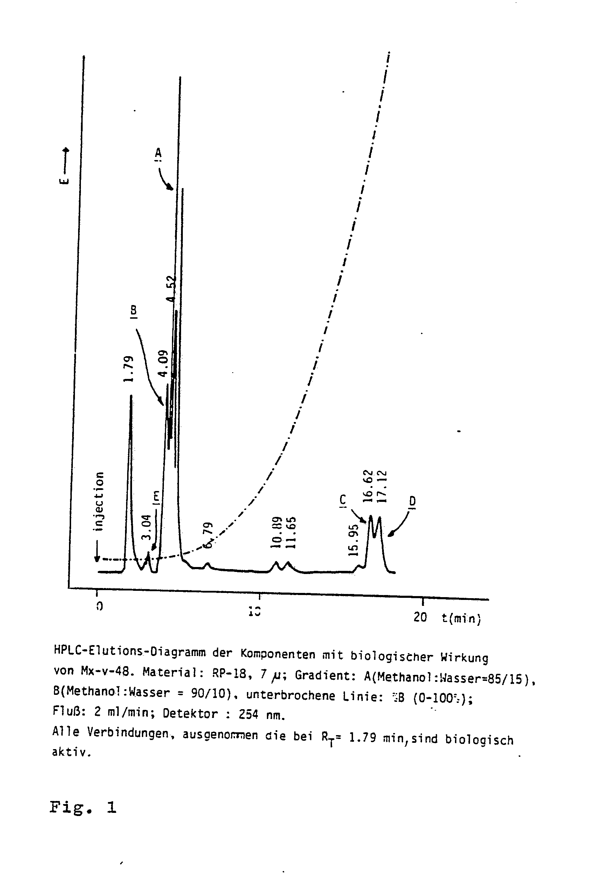

ein Wirkstoffgemisch gewinnen. Das Wirkstoffgemisch besteht aus mindestens zwölf Substanzen. Neben der UV-Löschung auf Kieselgel F$_{254}$ können die Wirkstoffe durch Einstellen in eine Jod-Kammer sichtbar gemacht werden. Nach Ansprühen der Wirkstoffe mit einer Lösung von Hydroxylamin/Eisen-(III)-chlorid bilden sich zunächst je nach Verbindung schwach orange bis rötlich gefärbte Flecken, letztere gehen langsam in eine violette Farbe über. Das HPLC-Elutions-Diagramm des Wirkstoffgemisches an Reversed-Phase-Material ist in Fig. 1 dargestellt. Die im folgenden beschriebene biologische Aktivität gegen E.coli wurde qualitativ bei allen Substanzen bis auf die mit der Retentionszeit R$_T$ = 1,79 nachgewiesen.

Die folgende Zusammenstellung zeigt das Wirkungsspektrum der Hauptkomponente A (bestehend aus dem Wirkstoff A1 und seinem Isomeren) im Plättchendiffusionstest. Die Konzentration des Gemisches betrug 10 µg/Plättchen. Die Aktivität wurde anhand des Hemmhofdurchmessers bestimmt. Die Testplatten wurden hergestellt durch Eingießen von 10 ml Agarmedium in Petrischalen von 9 cm Durchmesser. Der Agar wurde zuvor mit dem jeweiligen Testorganismus beimpft.

| Testorganismus | Hemmhofdurchmesser |
|---|---|
| Escherichia coli | 13 |
| Klebsiella spec. | 12 |
| Proteus mirabilis | 11 |
| Proteus morganii | 9,5 |
| Serratia marcescens | 12 |
| Salmonella typhimurium | 10 |
| Aerobacter aerogenes | 8 |
| Myxococcus virescens Mx-v-48 | 10 |
| Streptomyces heteropsis | 8 |
| Caryophanon latum | 18 |
| Azotobacter agilis | 11 |
| Nocardia flava | 14 |
| Alcaligenes eutrophus | 12 |
| Staphylococcus aureus | — |
| Bacillus subtilis | — |
| Bacillus polymyxa | — |
| Bacillus thuringiensis | — |
| Micrococcus lysodeikticus | — |
| Rhizobium meliloti | — |
| Pseudomonas acidovoran | — |
| Pseudomonas aeruginosa | — |
| Arthrobacter rubellus | — |
| Brevibacterium ammoniagenes | — |
| Vibrio extorquens | — |
| Agrobacterium tumefaciens | — |
| Candida albicans | — |
| Nadsonia fulvescens | — |
| Schizosaccharomyces pombe | — |
| Pichia membranaefaciens | — |
| Mucor hiemalis | — |
| Paecilomyces spec. | — |
| Penicillium digitatum | — |

**0 064 547**

Die minimale Hemmkonzentration (MHK) der Hauptkomponente A wurde im Reihenverdünnungstest ermittelt.

| Testorganismus | MHK ($\mu$g/ml) |
|---|---|
| Escherichia coli | 1 |
| Myxococcus virescens Mx-v-48 (DSM 1888) | 0,5 |
| Staphylococcus aureus | 25 |
| Candida albicans | 100 |

In Gegenwart von 10% Rinderserumalbumin erhöht sich die MHK gegenüber E.coli durch Bindung des Wirkstoffes an Albumin auf 3 $\mu$g/ml.

Die Protein- und DNA-Synthese wird bei Escherichia coli in Gegenwart von 4 $\mu$g der Hauptkomponente A innerhalb einer Stunde nur wenig gehemmt. Die RNS-Synthese wird gegenüber einer Kontrollkultur innerhalb einer Stunde um 40% gehemmt.

Charakterisierung einzelner Wirkstoffe (aus dem Gemisch von Mx-v-48 M8 = DSM 2177)

1. Hauptkomponente A, Wirkstoff A1 und sein Isomeres

Die die Hauptmenge des Wirkstoffgemisches bildende Komponente A kann aus dem Gemisch mittels Chromatographie an Reversed-Phase-Materialien isoliert werden. A besteht aus zwei isomeren Substanzen, aus denen A1 mittels franktionierter Kristallisation erhalten wird (Lösungsmittelgemisch: Hexan/Dichlormethan).

Chromatographie-Verhalten auf Kieselgel von A und A1

| Laufmittel-System | $R_f$-Wert |
|---|---|
| Essigsäureethylester | 0,1 |
| Dichlormethan/Methanol (15/1) | 0,43 |
| Hexan/Isopropanol (4/1) | 0,17 |
| Hexan/Aceton (1/1) | 0,14 |

Massenspektrum von A und A1 (70 eV, 230°C, MS 9):
$C_{35}H_{61}NO_8$ (Hochauflösung) gef.: 623,4395, ber.: 623,4397.

Das Molekül-Ion verliert Methanol und geht über in
$C_{34}H_{57}NO_7$ gef.: 591,4136, ber.: 591,4138.

Dieses Fragment-Ion verliert $C_5H_8O_2$ und geht über in
$C_{29}H_{49}NO_5$ gef.: 491,3610, ber.: 491,3610.

Optische Drehung von A1:
$alpha_D = +27,3°C$, c = 1 (Methanol)

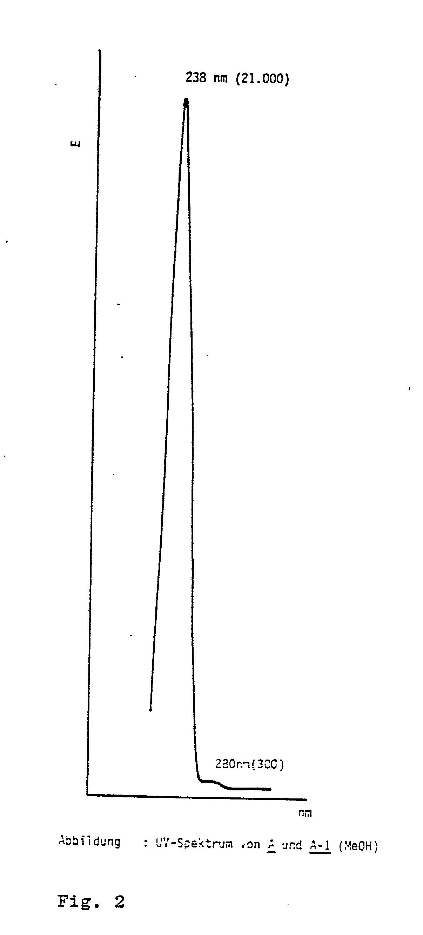

UV-Spektrum (Methanol; Fig. 2):
von A und A1; $lambda_{max} = 238$ nm (epsilon = 21 800), Schulter bei 280 nm (epsilon = 300).

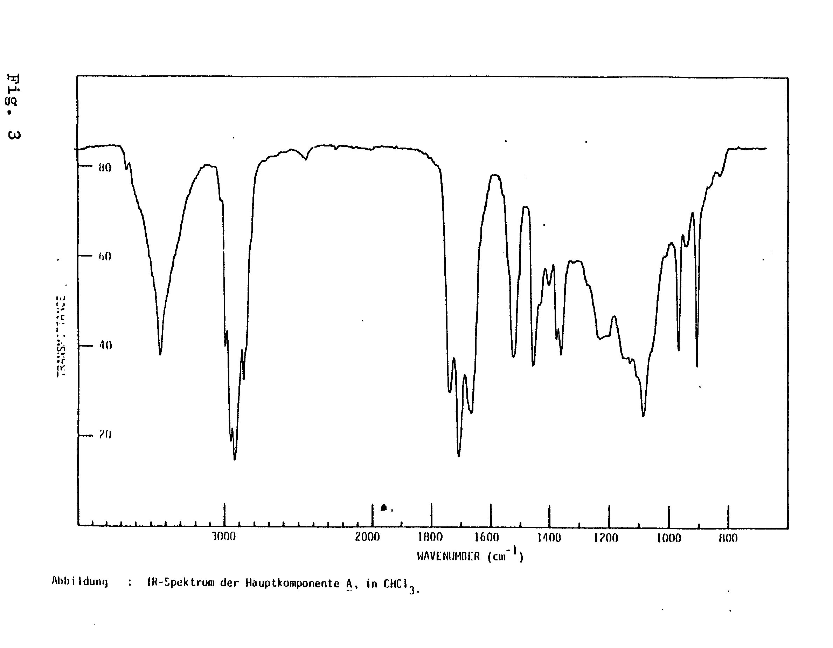

IR-Spektrum ($CHCl_3$; Fig. 3):
von A: ny = 3450, 3000, 2970, 2930, 2875, 1740, 1707, 1665, 1525, 1460, 1400, 1380, 1360, 1240, 1090, 970 und 908 cm$^{-1}$.

5

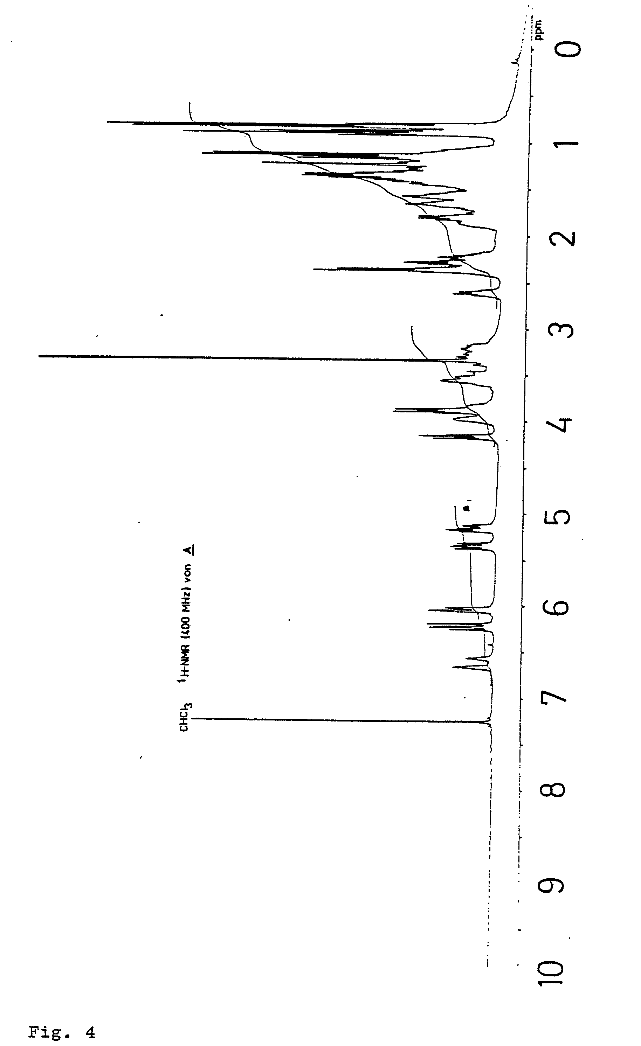

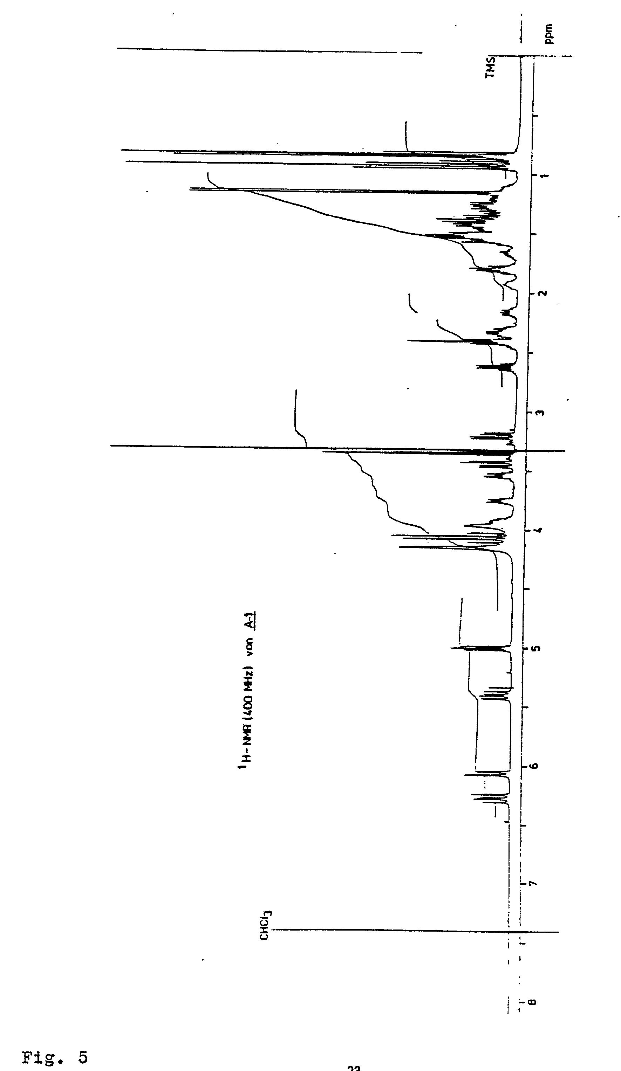

$^1$H—NMR-Spektrum (CDCl$_3$):
von A siehe Fig. 4, von A1 siehe Fig. 5.

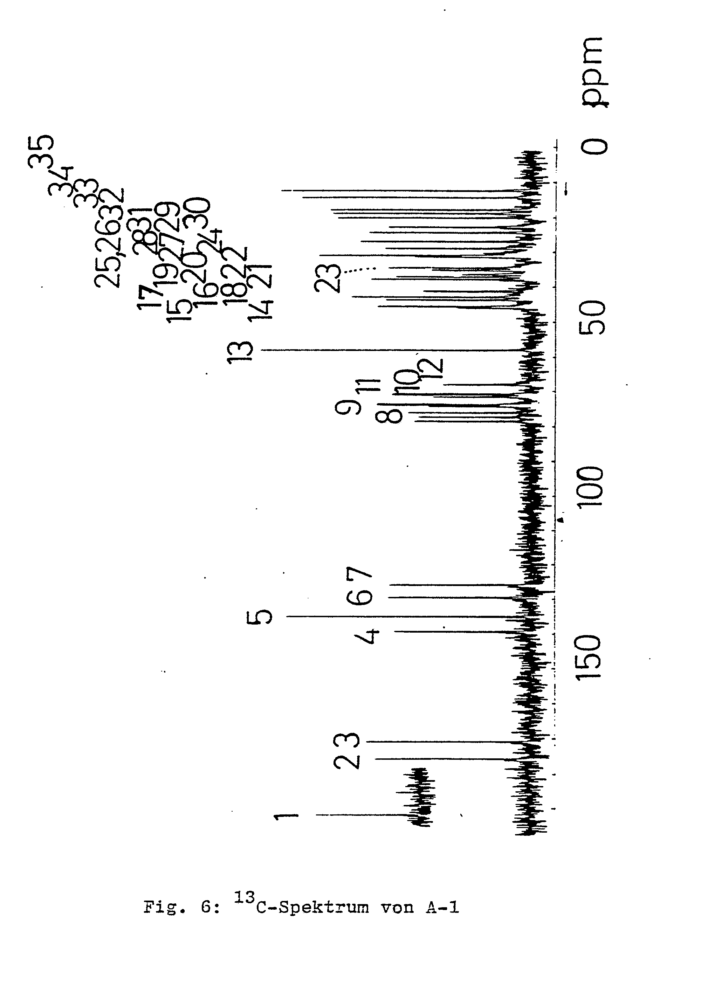

$^{13}$C—NMR-Spektrum (CDCl$_3$):
von A1 siehe Fig. 6.

Die chemischen Verschiebungen (delta, ppm) der entsprechend numerierten Kohlenstoffatome sind nachfolgend zusammengestellt:

| | | | | | | | | | |
|---|---|---|---|---|---|---|---|---|---|
| 1 = 212,2 | 8 = 74,0 | 15 = 44,9 | 22 = 34,4 | 29 = 23,6 |
| 2 = 176,1 | 9 = 73,5 | 16 = 43,0 | 23 = 33,8 | 30 = 22,0 |
| 3 = 171,1 | 10 = 71,2 | 17 = 42,1 | 24 = 30,6 | 31 = 19,4 |
| 4 = 139,2 | 11 = 70,5 | 18 = 40,6 | 25 = 30,2 | 32 = 18,1 |
| 5 = 134,9 | 12 = 67,7 | 19 = 37,0 | 26 = 30,2 | 33 = 17,1 |
| 6 = 129,3 | 13 = 57,7 | 20 = 36,3 | 27 = 28,2 | 34 = 13,5 |
| 7 = 125,7 | 14 = 45,3 | 21 = 35,7 | 28 = 26,2 | 35 = 11,7 |

## 2. Wirkstoff B

Massenspektrum:
Durch Hochauflösung wird die Summenformel zu C$_{35}$H$_{59}$NO$_8$ ermittelt. Gefunden wurde: 621,4257, für obige Summenformel berechnet: 621,4240. Das niederaufgelöste Massenspektrum zeigt die gleichen Fragmentierungen wie für A und A1:
M$^+$ —CH$_3$OH—H$_2$O—H$_2$O—H$_2$O.

UV-Spektrum (Methanol):
lambda$_{max}$ = 238 nm (epsilon = 20 000), 236 nm (epsilon = 20 200), 280 nm (epsilon = 300).

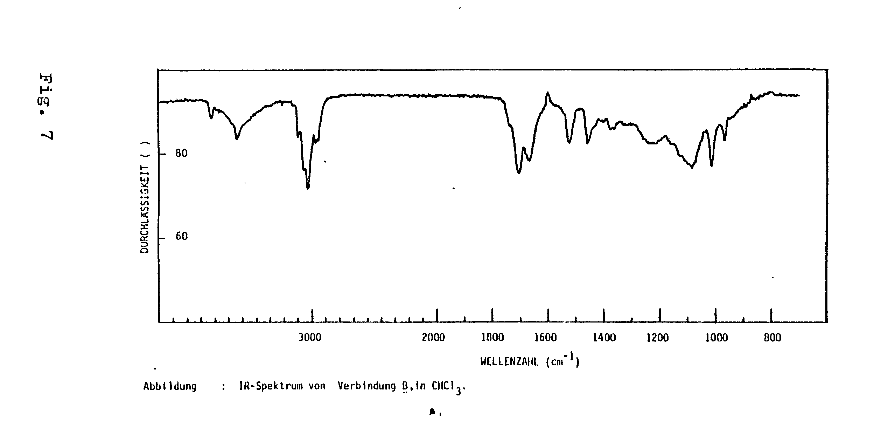

IR-Spektrum
siehe Fig. 7.

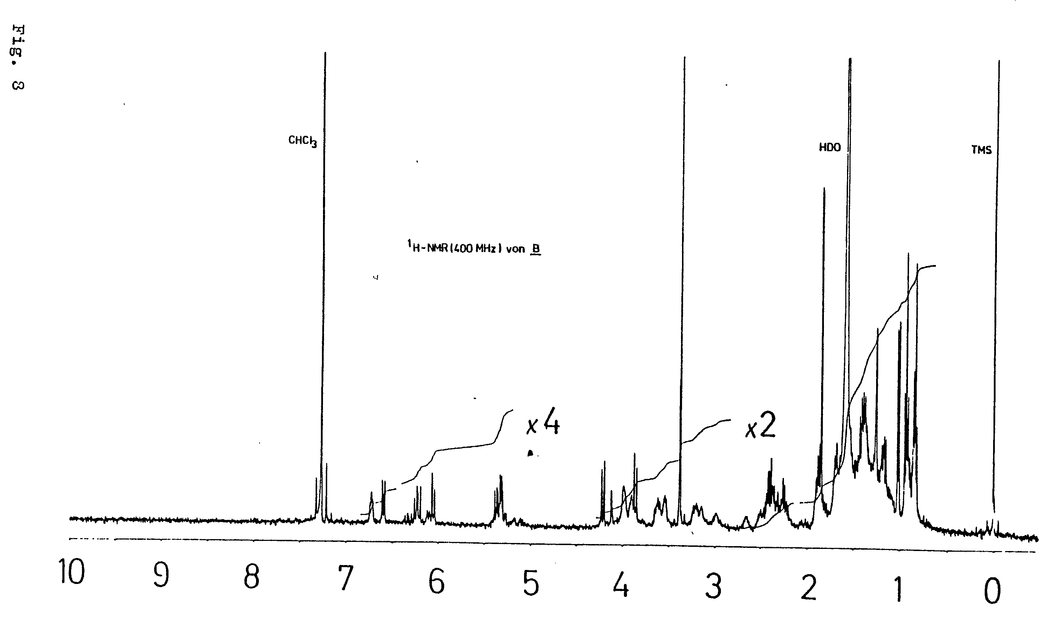

$^1$H—NMR-Spektrum
siehe Fig. 8.

Laufverhalten auf der Dünnschichtplatte (Kieselgel) im System Aceton : Hexan = 1 : 1:
R$_f$ = 0,12

## 3. Wirkstoffe C und D

Massenspektrum:
Die Summenformel C$_{35}$H$_{63}$NO$_7$ wird durch Hochauflösung ermittelt. Gef.: 609,4599, für die Summenformel berechnet: 609,4604. Die niederaufgelösten Massenspektren zeigen für beide Verbindungen ebenfalls die Fragmentierungen:
M$^+$ —CH$_3$OH—H$_2$O—H$_2$O—H$_2$O.

UV-Spektrum:
lambda$_{max}$ = 238 nm (epsilon = 21 000), keine Absorption bei 280 nm.

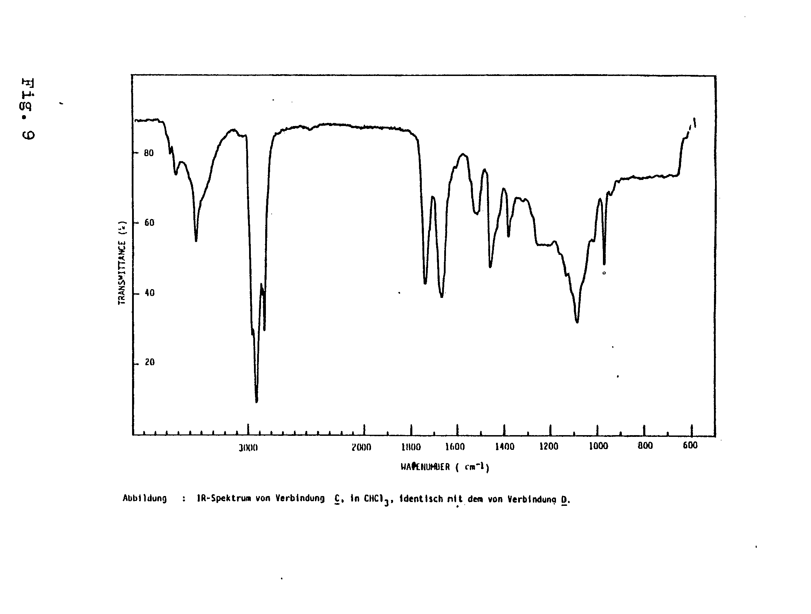

IR-Spektren:
siehe Fig. 9.

Optische Drehungen:
C: alpha$_D$ = +22,2° (c = 0,3, CHCl$_3$),
D: alpha$_D$ = + 6,3° (c = 0,25, CHCl$_3$).

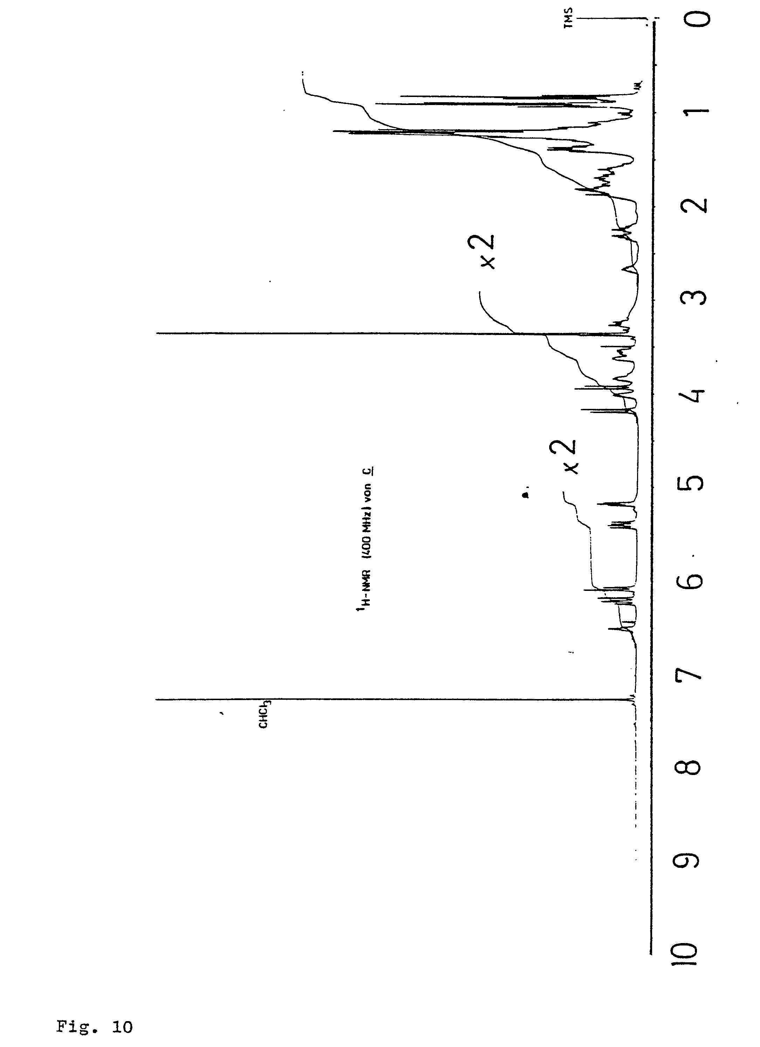

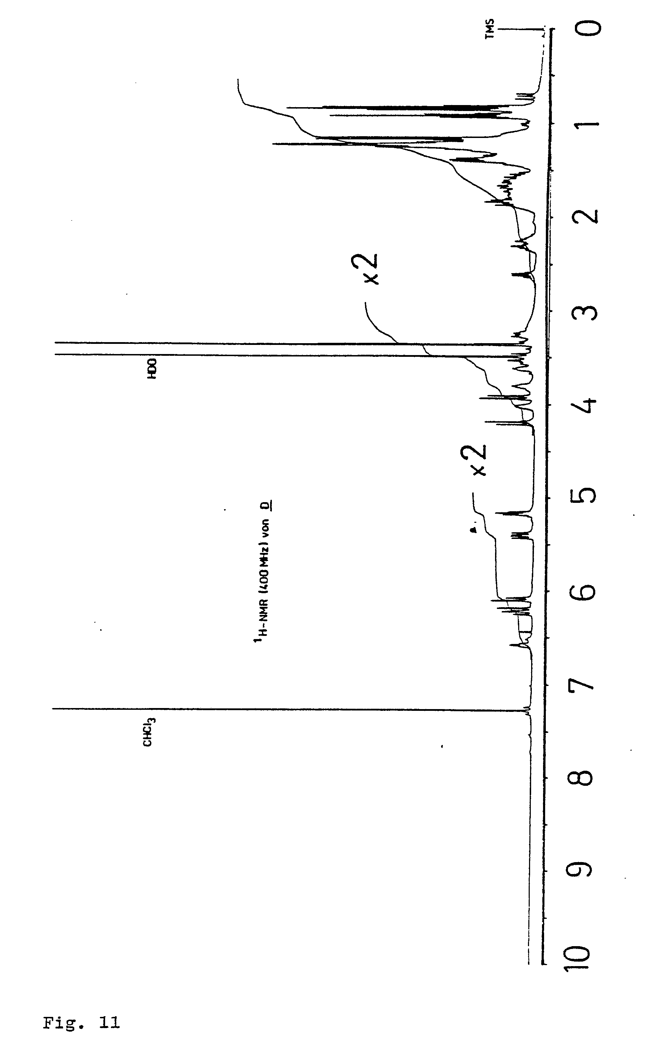

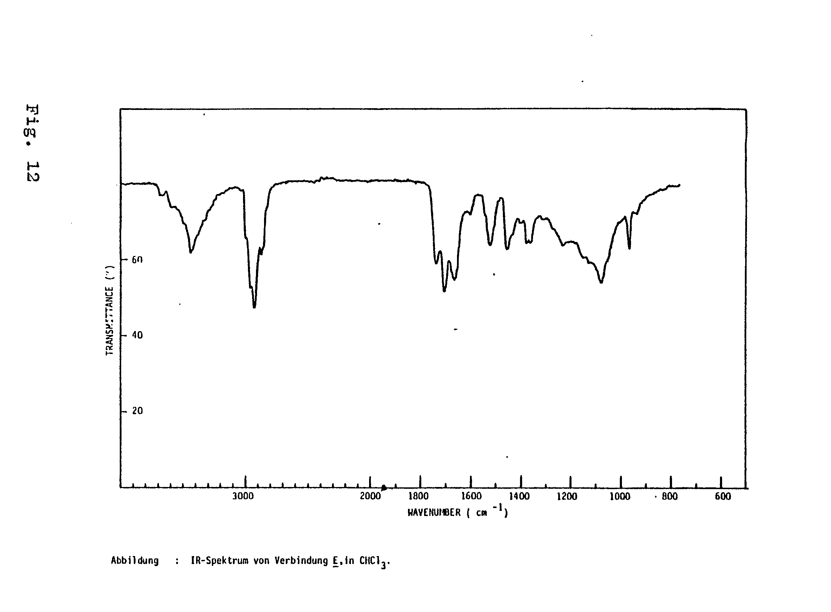

$^1$H—NMR-Spektrum
von C: siehe Fig. 10,
von D: siehe Fig. 11.

Laufverhalten (Kieselgel; Hexan : Aceton = 1 : 1):
von C: R$_f$ = 0,18,
von D: R$_f$ = 0,21.

## 4. Wirkstoff E

Für den Peak im Elutions-Diagramm (siehe Fig. 1) bei der Retentionszeit 3,04 sind zwei Substanzen verantwortlich. Die letzte von ihnen mit der angegebenen Zeit ließ sich präparativ abtrennen und zeigte die folgenden Eigenschaften.

Massenspektrum:
Durch Hochlauflösung wurde die Summenformel $C_{32}H_{55}NO_8$ bestimmt. Gef.: 581,39200, für die Formel ber.: 581,39274.
Das niederaufgelöste Spektrum erbrachte erneut die für A beschriebenen Fragmentierungen.

UV-Spektrum (Methanol):
$lambda_{max} = 238$ nm (epsilon $= 20\ 000$), 232 nm (epsilon $= 21\ 000$), 280 nm (epsilon $= 300$).

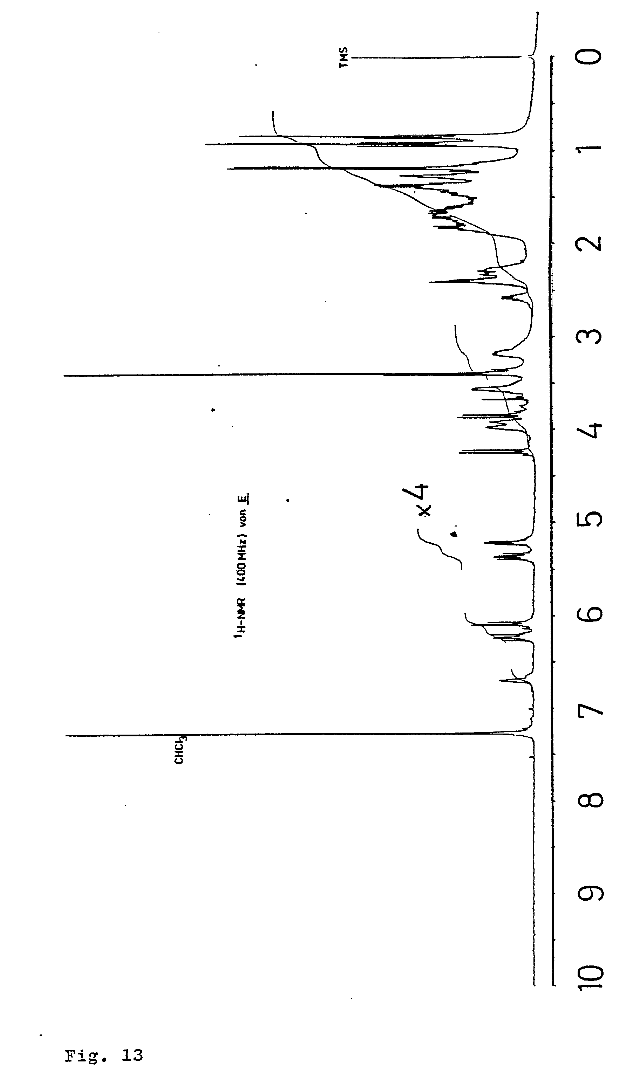

IR-Spektrum:
siehe Fig. 12.

$^1H-$NMR-Spektrum:
siehe Fig. 13.

Laufverhalten Hexan : Aceton $= 1 : 1$:
$R_f = 0,10$ (Kieselgel).

Nachstehend wird die Erfindung durch ein Beispiel näher erläutert.

### Beispiel

Fermentiert wird in folgendem Medium:

| | |
|---|---|
| 0,4% g/v | Corn Steep Pulver |
| 0,1% g/v | Sojabohnenmehl |
| 0,1% g/v | Pepton trypt. |
| 0,025% g/v | $MgSO_4 \cdot 7\,H_2O$ |
| 0,005% g/v | $CaCl_2 \cdot 2\,H_2O$ |
| 1 mg | $CoCl_2 \cdot 6\,H_2O/1$ |

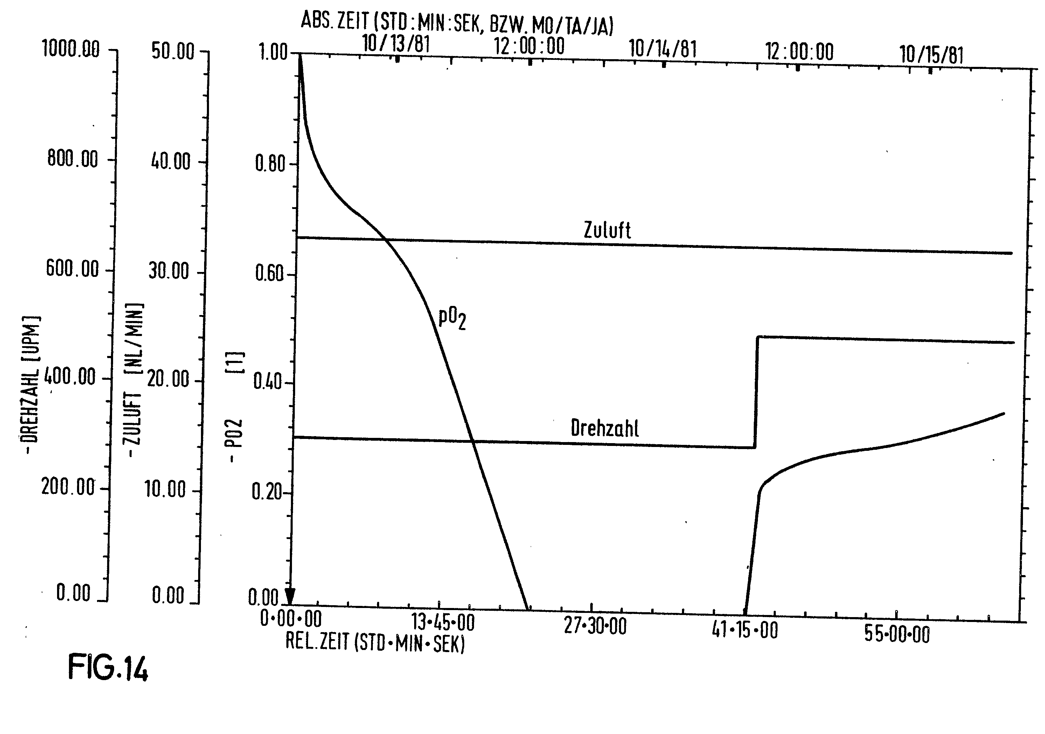

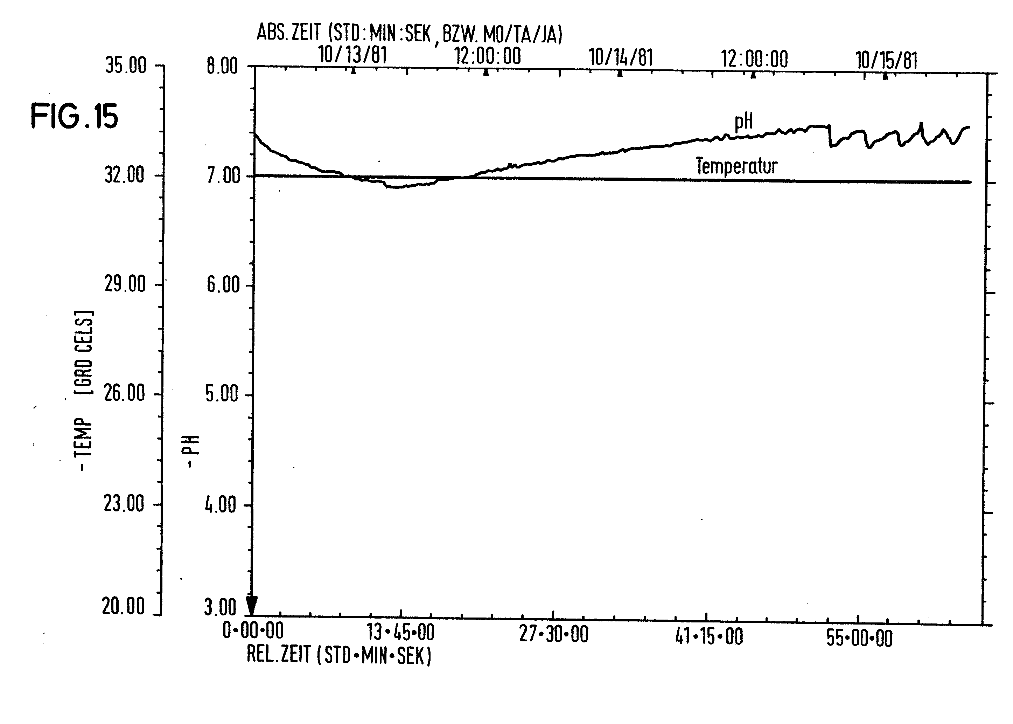

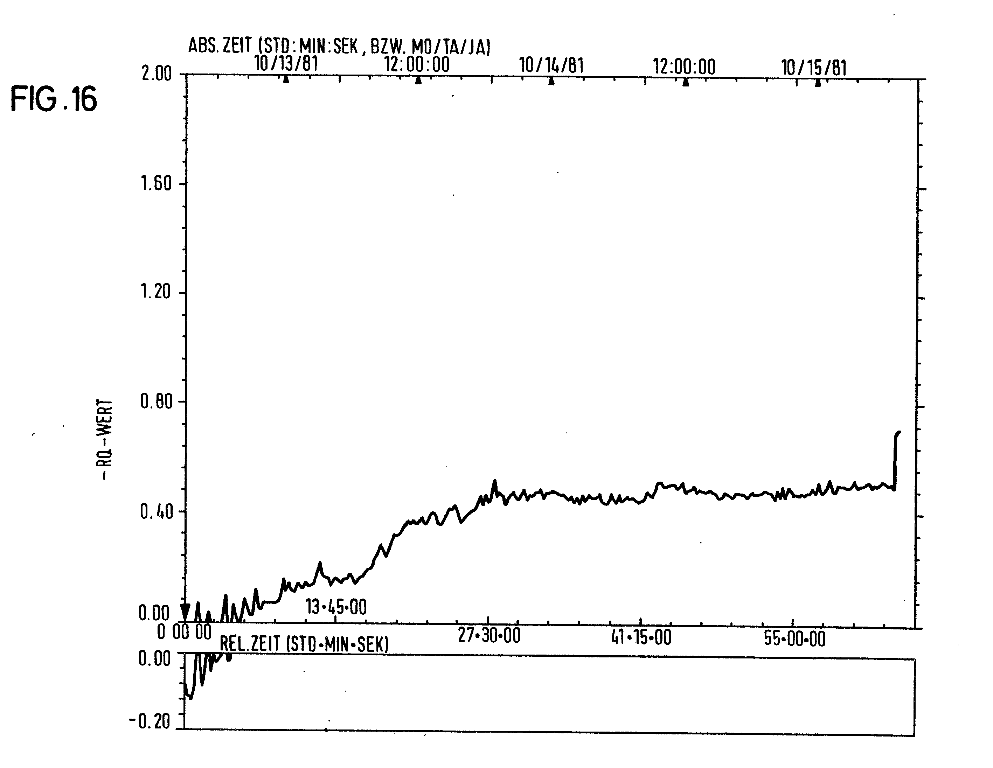

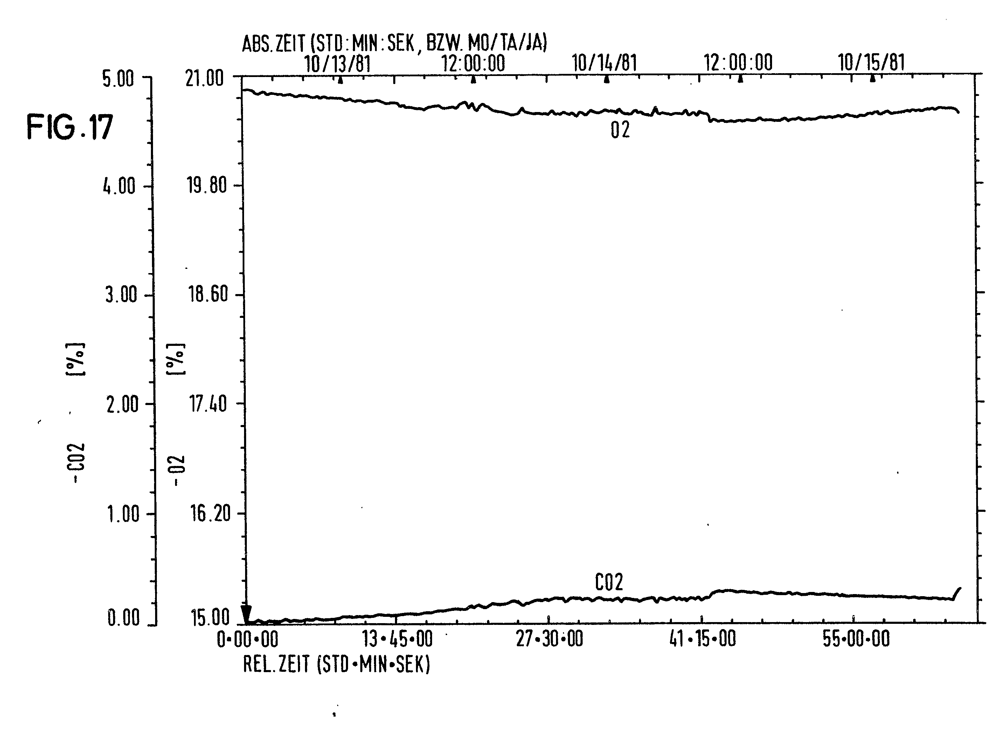

Der Verlauf einer typischen Fermentation des Stammes Mx-v-48 M8 (DSM 2177) ist in den Figuren 14 bis 17 abgebildet. Die Ausbeute an Wirkstoffen bezogen auf die Hauptkomponente A (bestehend aus dem Wirkstoff A1 und seinem Isomeren) betrug 2—2,5 mg im Durchschnitt. Die Fermentationen in 300-l- bzw. 700-l-Fermentern führte zu entsprechenden Ergebnissen.

Zur Gewinnung der Wirkstoffe wurden die Zellen vom Nährboden abzentrifugiert. Die Kulturbrühe wurde mittels einer Gegenstromextraktion mit Essigester extrahiert. Alternativ wurde die Extraktphase durch Einführen eines Adsorberharzes auf Polystyrolbasis (Amberlite XAD-2 oder XAD-4) in die abgetrennte Kulturbrühe und Elution des von der Brühe abgetrennten Adsorberharzes mittels Aceton erhalten. Der jeweilige Extrakt wurde entwässert, eingeengt und zwischen Methanol und Hexan zur Abtrennung von unpolaren, nichtaktiven Substanzen verteilt. Die Methanol-Phase wurde eingeengt und über Sephadex LH-20 chromatographiert. Aus einem 70-l-Fermenter wurden aus etwa 30 g Methanol-Inhaltsstoffen nach Chromatographie über Sephadex LH-20 etwa 600 mg eines biologisch-aktiven Substanzgemisches erhalten. Zur weiteren Reinigung wurde das erhaltene Rohgemisch an einer präparativen Säule (Waters-LC) mit Methanol und 10% Wasser als Laufmittel chromatographiert. Die Auftrennung in die einzelnen Fraktionen gelang unter Zuhilfenahme einer halbpräparativen Reversed-Phase-Säule (Knauer-Säule RP-18). Eluiert wurde mit einem Laufmittelgradienten, der in der Fig. 1 angegeben ist.

## Patentansprüche

1. Fester Wirkstoff A-1, gekennzeichnet durch folgende Parameter:

ein triklines Kristallgitter P1 mit den Gitterkonstanten a $= 14,412$ (6), b $= 22,650$ (6), c $= 6,238$ (3)Å, alpha $= 90,00$ (2), $\beta = 102,56$ (4), gamma $= 90,04$ (3) °, z $= 2$, $D_{ber} = 1,04$ g $\cdot$ cm$^3$;
einen FP. von 42 bis 43° C;
Nadeln aus Dichlormethan/n-Hexan-Gemischen;
UV-Löschung auf Kieselgel $F_{254}$;

den $R_f$-Werten bei Chromkatographie auf Kieselgel mit Essigsäureäthylester 0,1, mit Dichlormethan/ Methanol (15/1) 0,43, mit Hexan/Isopropanol (4/1) 0,17 und mit Hexan/Aceton (1/1) 0,14;
eine optische Drehung alpha$_D$ = +27,3°C, c = 1 (Methanol);
und die charakteristischen Spektren:

UV in Methanol (Figur 2) mit einem Wert von lambda$_{max}$ von 238 bei einem Extinktionskoeffizienten von 21 800 und einer Schulter bei 280 nm bei einem Extinktionskoeffizienten von 300;
ein Massenspektrum mit einem Molekülion-Peak für $C_{35}H_{61}NO_8$ und Fragmentionen-Peaks für $C_{34}H_{57}NO_7$ und $C_{29}H_{49}NO_5$;
ein IR-Spektrum in CHCl$_3$ (Figur 3) mit den Werten für ny = 3450, 3000, 2970, 2930, 2875, 1740, 1707, 1665, 1525, 1460, 1400, 1380, 1360, 1240, 1090, 970 und 908 cm$^{-1}$;
ein $^1$H—NMR-Spektrum in CDCl$_3$ gemäß Figur 5;
ein $^{13}$C—NMR-Spektrum in CDCl$_3$ gemäß Figur 6 mit den chemischen Verschiebungen (delta, ppm) der entsprechend numerierten C-Atome (Multiplizitäten im Off-Resonanz-Spektrum: s = Singulett, d = Dublett, t = Triplett, q = Quartett):

| | | | | | | |
|---|---|---|---|---|---|---|
| 1 | = | 212,2 s C—CO—C | | 19 | = | 37,0 d —CH< |
| 2 | = | 176,1 s C—CO—O | | 20 | = | 36,3 t |
| 3 | = | 171,1 s C—CO—NH | | 21 | = | 35,7 t |
| 4 | = | 139,2 d =CH— | | 22 | = | 34,4 t |
| 5 | = | 134,9 s =C< | | 23 | = | 33,8 t |
| 6 | = | 129,3 d =CH— | | 24 | = | 30,6 t |
| 7 | = | 125,7 d =CH— | | 25 | = | 30,2 d —CH< |
| 8 | = | 74,0 d >CH—O | | 26 | = | 30,2 t |
| 9 | = | 73,5 d >CH—O | | 27 | = | 28,2 t |
| 10 | = | 71,2 d >CH—O | | 28 | = | 26,2 t |
| 11 | = | 70,5 t —CH$_2$—OCH$_3$ | | 29 | = | 23,6 t |
| 12 | = | 67,7 d >CH—O | | 30 | = | 22,0 t |
| 13 | = | 57,7 q O—CH$_3$ | | 31 | = | 19,4 q CH$_3$ |
| 14 | = | 45,3 t | | 32 | = | 18,1 t |
| 15 | = | 44,9 d —CH< | | 33 | = | 17,1 q CH$_3$ |
| 16 | = | 43,0 t | | 34 | = | 13,5 q CH$_3$ |
| 17 | = | 42,1 t | | 35 | = | 11,7 q CH$_3$ |
| 18 | = | 40,6 t | | | | |

und durch Abtrennbarkeit aus einem Wirkstoffgemisch gemäß Anspruch 8, und sein Isomeres.
   2. Fester Wirkstoff B, gekennzeichnet durch folgende Parameter:
den $R_f$-Wert bei Chromatographie auf Kieselgel mit Aceton/Hexan (1/1) 0,12, und die charakteristischen Spektren:

UV in Methanol mit einem Wert von lambda$_{max}$ von 280 bei einem Extinktionskoeffizienten von 300, 230 bei einem Extinktionskoeffizienten von 40 000 und
einem Massenspektrum entsprechend der Summenformel $C_{35}H_{59}NO_8$;
einem IR-Spektrum gemäß Figur 7 und
einem $^1$H—NMR-Spektrum gemäß Figur 8 und

durch Abtrennbarkeit aus einem Wirkstoffgemisch gemäß Anspruch 8.
   3. Fester Wirkstoff C, gekennzeichnet durch folgende Parameter:
den $R_f$-Wert bei Chromatographie auf Kieselgel mit Hexan/Aceton (1/1) 0,18;
eine optische Drehung alpha$_D$ = +22,2°, c = 0,3 (Chloroform) und die charakteristischen Spektren:

UV mit einem Wert von lambda$_{max}$ von 238 nm bei einem Extinktionskoeffizienten von 21 000 (keine Absorption bei 280 nm);
einem Massenspektrum entsprechend der Summenformel $C_{35}H_{63}NO_7$;
einem IR-Spektrum gemäß Figur 9 und
einem $^1$H—NM:R-Spektrum gemäß Figur 10 und

durch Abtrennbarkeit aus einem Wirkstoffgemisch gemäß Anspruch 8.
   4. Fester Wirkstoff D, gekennzeichnet durch folgende Parameter:
den $R_f$-Wert bei Chromatographie auf Kieselgel mit Hexan/Aceton (1/1) 0,21;
eine optische Drehung alpha$_D$ = +6,3°, c = 0,25 (Chloroform) und die charakteristischen Spektren:

**0 064 547**

UV mit einem Wert von lambda$_{max}$ von 238 nm bei einem Extinktionskoeffizienten von 21 000 (keine Absorption bei 280 nm);
einem Massenspektrum entsprechend der Summenformel $C_{35}H_{63}NO_7$;
einem IR-Spektrum gemäß Figur 9 und
einem $^1H-$NMR-Spektrum gemäß Figur 11 und

durch Abtrennbarkeit aus einem Wirkstoffgemisch gemäß Anspruch 8.

5. Fester Wirkstoff E, gekennzeichnet durch folgende Parameter:
den $R_f$-Wert bei Chromatographie auf Kieselgel mit Hexan/Aceton (1/1) 0,10 und
die charakteristischen Spektren:

UV in Methanol mit einem Wert von lambda$_{max}$ von 280 bei einem Extinktionskoeffizienten von 300, 238 bei einem Extinktionskoeffizienten von 20 000 und 232 nm bei einem Extinktionskoeffizienten von 21 000;
einem Massenspektrum entsprechend der Summenformel $C_{32}H_{55}NO_8$;
einem IR-Spektrum gemäß Figur 12 und
einem $^1H-$NMR-Spektrum gemäß Figur 13 und

durch Abtrennbarkeit aus einem Wirkstoffgemisch gemäß Anspruch 8.

6. Wirkstoffe 1 bis 5, gekennzeichnet durch folgende Parameter:

| Wirkstoff | Retentionszeit (min) |
|-----------|----------------------|
| 1 | ca. 2,9 |
| 2 | 6,79 |
| 3 | 10,89 |
| 4 | 11,65 |
| 5 | 15,95 |

im HPLC-Elutionsdiagramm gemäß Figur 1 (RP-18; 7 μm; Gradient: A (Methanol/Wasser = 85/15), B (Methanol/Wasser = 90/10); unterbrochene Linie: B (%) = 0 bis 100%; Fluß: 2 ml/min; Detektor: 254 nm)
und durch Abtrennbarkeit aus einem Wirkstoffgemisch gemäß Anspruch 8.

7. Verfahren zur Herstellung eines Wirkstoffes gemäß einem der Ansprüche 1 bis 6, dadurch gekennzeichnet, daß man

(I) Myxococcus virescens DSM 1888 oder DSM 2177 in einem Kohlenstoff und Stickstoff sowie Mineralsalze enthaltenden wässerigen Medium bei etwa 15 bis 40° C kultiviert,
(II) die gesamte Kulturbrühe (gegebenenfalls nach einem Aufschluß der Zellen von Myxococcus virescens DSM 1888 oder DSM 2177), die von den Zellen von Myxococcus virescens DSM 1888 oder 2177 abgetrennte Kulturbrühe oder die von der Kulturbrühe abgetrennten Zellen von Myxococcus virescens DSM 1888 oder DSM 2177 (gegebenenfalls nach einem Aufschluß der Zellen)
   (a) mit Essigsäureäthylester extrahiert und die Extraktphase abtrennt oder
   (b) mit einem Abdsorberharz auf Polystyrolbasis extrahiert, das Adsorberharz mit Aceton eluiert und die Extraktphase abtrennt,
(III) von der Extraktphase
   (a) das Aceton bzw. den Essigsäureäthylester abdestilliert,
   (b) (vor oder nach dem Abdestillieren des organischen Lösungsmittels) den erhaltenen Rückstand trocknet,
   (c) den Rückstand zwischen einer polaren und einer unpolaren organischen Phase verteilt,
   (d) die polare organische Phase einengt,
   (e) die eingeengte polare organische Phase beispielsweise über Sephadex LH-20 chromatographiert und
   (f) die Laufmittelphase einengt und
(IV) das erhaltene Wirkstoffgemisch an einer mit Reversed-Phase-Kieselgel beschickten Säule mit einem polaren organischen Lösungsmittel unter Zusatz von Wasser chromatographiert, den jeweiligen Wirkstoff fraktioniert abtrennt und durch Abdampfen des Laufmittels vor oder nach dem Trocknen und gegebenenfalls durch fraktionierte Kristallisation isoliert.

9

8. Wirkstoffgemisch erhältlich durch die Maßnahmen gemäß Anspruch 7, Stufen (I) bis (III).

9. Mikroorganismus Myxococcus virescens DSM 1888.

10. Mikroorganismus Myxococcus virescens DSM 2177.

**Claims**

1. Solid active substance A-1, characterised by the following parameters:

a triclinic crystal lattice P1 having the lattice constants a = 14.412 (6), b = 22.650 (6), c = 6.238 (3) Å, alpha = 90.00 (2), $\beta$ = 102.56 (4), gamma = 90.04 (3) °, z = 2, $D_{calc.}$ = 1.04 g · $cm^3$;
a melting point of from 42 to 43° C;
needles comprising dichloromethane/n-hexane mixtures;
UV extinction over silica gel $F_{254}$;
$R_f$ values for chromatography over silica gel of 0.1 using ethyl acetate, of 0.43 using dichloromethane/methanol (15/1), of 0.17 using hexane/isopropanol (4/1) and of 0.14 using hexane/acetone (1/1);
an optical rotation of $alpha_D$ = + 27.3°, c = 1 (methanol);
and by the characteristic spectra:

UV in methanol (Figure 2) with a value of $lambda_{max}$ of 238 at a coefficient of extinction of 21,800 and a shoulder at 280 nm at a coefficient of extinction of 300;
a mass spectrum having a molecular ion peak for $C_{35}H_{61}NO_8$ and fragment ion peaks for $C_{34}H_{57}NO_7$ and $C_{29}H_{49}NO_5$;
an IR spectrum in $CHCl_3$ (Figure 3) with values for $\nu$ = 3450, 3000, 2970, 2930, 2875, 1740, 1707, 1665, 1525, 1460, 1400, 1380, 1360, 1240, 1090, 970 and 908 $cm^{-1}$;
a $^1H$—NMR spectrum in $CDCl_3$ according to Figure 5;
a $^{13}C$—NMR spectrum in $CDCl_3$ according to Figure 6 with the chemical shifts (delta, ppm) of the correspondingly numbered carbon atoms (multiplicities in the off-resonance spectrum: s = singlet, d = doublet, t = triplet, q = quartet):

| | | | | |
|---|---|---|---|---|
| 1 = | 212.2 s C—CO—C | | 19 = | 37.0 d —CH< |
| 2 = | 176.1 s C—CO—O | | 20 = | 36.3 t |
| 3 = | 171.1 s C—CO—NH | | 21 = | 35.7 t |
| 4 = | 139.2 d =CH— | | 22 = | 34.4 t |
| 5 = | 134.9 s =C< | | 23 = | 33.8 t |
| 6 = | 129.3 d =CH— | | 24 = | 30.6 t |
| 7 = | 125.7 d =CH— | | 25 = | 30.2 d —CH< |
| 8 = | 74.0 d >CH—O | | 26 = | 30.2 t |
| 9 = | 73.5 d >CH—O | | 27 = | 28.2 t |
| 10 = | 71.2 d >CH—O | | 28 = | 26.2 t |
| 11 = | 70.5 t —CH₂—OCH₃ | | 29 = | 23.6 t |
| 12 = | 67.7 d >CH—O | | 30 = | 22.0 t |
| 13 = | 57.7 q O—CH₃ | | 31 = | 19.4 q CH₃ |
| 14 = | 45.3 t | | 32 = | 18.1 t |
| 15 = | 44.9 d —CH< | | 33 = | 17.1 q CH₃ |
| 16 = | 43.0 t | | 34 = | 13.5 q CH₃ |
| 17 = | 42.1 t | | 35 = | 11.7 q CH₃ |
| 18 = | 40.6 t | | | |

and by an ability to be separated off from a mixture of active substances according to claim 8, and the isomer thereof.

2. Solid active substance B, characterised by the following parameters:
an $R_f$ value for chromatography over silica gel using acetone/hexane (1/1) of 0.12; and
by the characteristic spectra:

UV in methanol with a value of $lambda_{max}$ of 280 at a coefficient of extinction of 300 and 230 at a coefficient of extinction of 40,000, and
a mass spectrum corresponding to the empirical formula $C_{35}H_{59}NO_8$;
an IR spectrum according to Figure 7; and
a $^1H$—NMR spectrum according to Figure 8; and

by an ability to be separated off from a mixture of active substances according to claim 8.

3. Solid active substance C, characterised by the following parameters:
an $R_f$ value for chromatography over silica gel using hexane/acetone (1/1) of 0.18;
an optical rotation of $alpha_D$ = + 22.2°, c = 0.3 (chloroform) and

by the characteristic spectra:

UV with a value of lambda$_{max}$ of 238 nm at a coefficient of extinction of 21,000 (no absorption at 280 nm);
a mass spectrum corresponding to the empirical formula $C_{35}H_{63}NO_7$;
an IR spectrum according to Figure 9; and
a $^1H-NMR$ spectrum according to Figure 10; and

by an ability to be separated off from a mixture of active substances according to claim 8.

4. Solid active substance D, characterised by the following parameters:
an $R_f$ value for chromatography over silica gel using hexane/acetone (1/1) of 0.21;
an optical rotation of alpha$_D$ = +6.3°, c = 0.25 (chloroform); and
by the characteristic spectra:

UV with a value of lambda$_{max}$ of 238 nm at a coefficient of extinction of 21,000 (no absorption at 280 nm);
a mass spectrum corresponding to the empirical formula $C_{35}H_{63}NO_7$;
an IR spectrum according to Figure 9 and
a $^1H-NMR$ spectrum according to Figure 11 and

by an ability to be separated off from a mixture of active substances according to claim 8.

5. Solid active substance E, characterised by the following parameters:
an $R_f$ value for chromatography over silica gel using hexane/acetone (1/1) of 0.10 and
by the characteristic spectra:

UV in methanol with a value of lambda$_{max}$ of 280 at a coefficient of extinction of 300, of 238 at a coefficient of extinction of 20,000 and of 232 at a coefficient of extinction of 21,000;
a mass spectrum corresponding to the empirical formula $C_{32}H_{55}NO_8$;
an IR spectrum according to Figure 12 and
a $^1H-NMR$ spectrum according to Figure 13 and

by an ability to be separated off from a mixture of active substances according to claim 8.

6. Active substances 1 to 5, characterised by the following parameters:

| Active substance | Retention time (min) |
| --- | --- |
| 1 | approx. 2.9 |
| 2 | 6.79 |
| 3 | 10.89 |
| 4 | 11.65 |
| 5 | 15.95 |

in the HPLC elution diagram according to Figure 1 (RP-18; 7 µm; gradient: A (methanol/water = 85/15), B (methanol/water = 90/10); broken line: B (%) = from 0 to 100%, flow: 2 ml/min; detector: 254 nm) and by an ability to be separated off from a mixture of active substances according to claim 8.

7. Process for the manufacture of an active substance according to any one of claims 1 to 6, characterised in that

(I)   Myxococcus virescens DSM 1888 or DSM 2177 is cultivated in an aqueous medium containing carbon and nitrogen and mineral salts at approximately from 15 to 40° C,

(II)  the entire culture broth (if appropriate after decomposition of the cells of Myxococcus virescens DSM 1888 or DSM 2177), or the culture broth which has been separated off from the cells of Myxococcus virescens DSM 1888 or 2177, or the cells of Myxococcus virescens DSM 1888 or DSM 2177 which have been separated off from the culture broth (if appropriate after decomposition of the cells), is
      (a)  extracted with ethyl acetate and the extract phase is separated off, or
      (b)  extracted with an adsorber resin based on polystyrene, the adsorber resin being eluted with acetone and the extract phase being separated off,

(III) from the extract phase
- (a) the acetone or the ethyl acetate is distilled off,
- (b) (before or after the organic solvent is distilled off) the resulting residue is dried,
- (c) the residue is partitioned between a polar and an apolar organic phase,
- (d) the polar organic phase is concentrated,
- (e) the concentrated polar organic phase is chromatographed, for example over Sephadex LH-20, and
- (f) the eluant phase is concentrated, and

(IV) the resulting mixture of active substances is chromatographed over a column charged with reversed-phase silica gel using a polar organic solvent with the addition of water, and the individual active substances are separated off fractionally and isolated by evaporating off the eluant before or after drying and if appropriate by means of fractional crystallisation.

8. Mixture of active substances obtainable by the measures according to claim 7, stages (I) to (III).

9. Micro-organism Myxococcus virescens DSM 1888.

10. Micro-organism Myxococcus virescens DSM 2177.


## Revendications

1. Substance active solide A-1 caractérisée par les paramètres suivants:

— réseau cristallin triclinique P1 présentant les constantes de réseau suivantes:
$a = 14,412 (6)$, $b = 22,650 (6)$, $c = 6,238 (3)$ Å,
$\alpha = 90,00 (2)$, $\beta = 102,56 (4)$, $\gamma = 90,04 (3)$ °, $z = 2$, $D_{cal} = 1,04$ g/cm³;
— point de fusion de 42 à 43° C;
— aiguilles par cristallisation dans des mélanges de dichlorométhane et de n-hexane;
— extinction UV sur gel de silice $F_{254}$;
— valeurs $R_f$ dans la chromatographie sur gel de silice

| | |
|---|---|
| avec l'acétate d'éthyle: | 0,1 |
| avec dichlorométhane/méthanol (15/1): | 0,43 |
| avec hexane/isopropanol (4/1): | 0,17 |
| et avec hexane/acétone (1/1): | 0,14; |

— pouvoir rotatoire: $\alpha_D = +27,3°$, $c = 1$ (méthanol);
— et caractéristiques spectrales suivantes:

spectre ultraviolet dans le méthanol (figure 2): $\lambda_{max} = 238$ avec un coefficient d'extinction de 21 800, et épaulement à 280 nm avec un coefficient d'extinction de 300;
spectre de masse: pic d'ion moléculaire pour $C_{35}H_{61}NO_8$ et pics d'ions fragmentaires pour $C_{34}H_{57}NO_7$ et $C_{29}H_{49}NO_5$;
spectre infrarouge dans $CHCl_3$ (figure 3): $\nu = 3450, 3000, 2970, 2930, 2875, 1740, 1707, 1665, 1525, 1460, 1400, 1380, 1360, 1240, 1090, 970$ et $908$ cm⁻¹;
spectre de RMN ¹H dans $CDCl_3$: tel que représenté sur la figure 5;
spectre de RMN ¹³C dans $CDCl_3$: tel que représenté sur la figure 6, avec les déplacements chimiques suivants ($\delta$, ppm) des atomes C numérotés (multiplicités sur le spectre de off-résonance: s = singulet, d = doublet, t = triplet, q = quartet):

| | | | | |
|---|---|---|---|---|
| 1 = | 212,2 s C—CO—C | | 19 = | 37,0 d —CH< |
| 2 = | 176,1 s C—CO—O | | 20 = | 36,3 t |
| 3 = | 171,1 s C—CO—NH | | 21 = | 35,7 t |
| 4 = | 139,2 d =CH— | | 22 = | 34,4 t |
| 5 = | 134,9 s =C< | | 23 = | 33,8 t |
| 6 = | 129,3 d =CH— | | 24 = | 30,6 t |
| 7 = | 125,7 d =CH— | | 25 = | 30,2 d —CH< |
| 8 = | 74,0 d >CH—O | | 26 = | 30,2 t |
| 9 = | 73,5 d >CH—O | | 27 = | 28,2 t |
| 10 = | 71,2 d >CH—O | | 28 = | 26,2 t |
| 11 = | 70,5 t —CH₂—OCH₃ | | 29 = | 23,6 t |
| 12 = | 67,7 d >CH—O | | 30 = | 22,0 t |
| 13 = | 57,7 q O—CH₃ | | 31 = | 19,4 q CH₃ |
| 14 = | 45,3 t | | 32 = | 18,1 t |
| 15 = | 44,9 d —CH< | | 33 = | 17,1 q CH₃ |
| 16 = | 43,0 t | | 34 = | 13,5 q CH₃ |
| 17 = | 42,1 t | | 35 = | 11,7 q CH₃ |
| 18 = | 40,6 t | | | |

et par le fait qu'elle peut être séparée à partir d'un mélange de substances actives selon la revendication 8,

et isomère de cette substance.

2. Substance active solide B caractérisée par les paramètres suivants:

— valeur $R_f$ dans la chromatographie sur gel de silice avec acétone/hexane (1/1): 0,12;
— caractéristiques spectrales suivantes:
spectre UV dans le méthanol: $\lambda_{max}$ de 280 avec un coefficient d'extinction de 300, et de 230 avec un coefficient d'extinction de 40.000,
spectre de masse correspondant à la formule brute $C_{35}H_{59}NO_8$;
spectre infrarouge: selon la figure 7;
spectre de RMN $^1H$: selon la figure 8;

et en ce qu'elle peut être séparée à partir d'un mélange de substances actives selon la revendication 8.

3. Substance active solide C caractérisé par les paramètres suivants:

— valeur $R_f$ dans la chromatographie sur gel de silice avec hexane/acétone (1/1): 0,18;
— pouvoir rotatoire: $\alpha_D = +22,2°$, c = 0,3 (chloroforme);
— caractéristiques spectrales suivantes:

spectre UV: $\lambda_{max}$ de 238 nm avec un coefficient d'extinction de 21.000 (pas d'absorption à 280 nm);
spectre de masse correspondant à la formule brute $C_{35}H_{63}NO_7$;
spectre infrarouge: selon la figure 9;
spectre de RMN $^1H$: selon la figure 10;

et en ce qu'elle peut être séparée à partir d'un mélange de substances actives selon la revendication 8.

4. Substance active solide D caractérisée par les paramètres suivants:

— valeur $R_f$ dans la chromatographie sur gel de silice avec hexane/acétone (1/1): 0,21;
— pouvoir rotatoire: $\alpha_D = +6,3°$, c = 0,25 (chloroforme);
— caractéristiques spectrales suivantes:

spectre UV: $\lambda_{max}$ de 238 nm avec un coefficient d'extinction de 21.000 (pas d'absorption à 280 nm);
spectre de masse correspondant à la formule brute $C_{35}H_{63}NO_7$;
spectre infrarouge: selon la figure 9;
spectre de RMN $^1H$: selon la figure 11;

et en ce qu'elle peut être séparée à partir d'un mélange de substances actives selon la revendication 8.

5. Substance active solide E caractérisée par les paramètres suivantes:

— valeur $R_f$ dans la chromatographie sur gel de silice avec hexane/acétone (1/1): 0,10,
— caractéristiques spectrales suivantes:

spectre UV dans le méthanol: $\lambda_{max}$ de 280 avec un coefficient d'extinction de 300, de 238 avec un coefficient d'extinction de 20.000 et de 232 nm avec un coefficient d'extinction de 21.000;
spectre de masse correspondant à la formule brute $C_{32}H_{55}NO_8$;
spectre infrarouge: selon la figure 12;
spectre de RMN $^1H$: selon la figure 13;

et en ce qu'elle peut être séparée à partir d'un mélange de substances actives selon la revendication 8.

6. Substances actives 1 à 5 caractérisées par les paramètres suivants:

| Substance active | Temps de rétention (en minutes) |
|---|---|
| 1 | environ 2,9 |
| 2 | 6,79 |
| 3 | 10,89 |
| 4 | 11,65 |
| 5 | 15,95 |

13

dans le diagramme d'élution HPLC selon la figure 1 (RP-18; 7 µm; gradient: A (méthanol: eau = 85/15), B (méthanol/eau = 90/10); ligne discontinue: B (%) = 0 à 100%; écoulement: 2 ml/minute; détecteur: 254 nm),

et en ce qu'elles peuvent être séparées à partir d'un mélange de substances actives selon la revendication 8.

7. Procédé de préparation d'une substance active selon l'une quelconque des revendications 1 à 6, procédé caractérisé en ce que:

(I)    on cultive Myxococcus virescens DSM 1888 ou DSM 2177 dans un milieu aqueux contenant du carbone et de l'azote ainsi que des sels minéraux, à une température d'environ 15 à 40° C,

(II)   sur le bouillon de culture complet (éventuellement après désagrégation des cellules de Myxococcus virescens DSM 1888 ou DSM 2177), sur le bouillon de culture dont on a séparé les cellules de Myxococcus virescens DSM 1888 ou DSM 2177, ou sur les cellules de Myxococcus virescens DSM 1888 ou DSM 2177 qui ont été séparées du bouillon de culture (éventuellement après une désagrégation des cellules), on effectue les opérations suivantes:
    (a)   on extrait par de l'acétate d'éthyle et on sépare la phase d'extrait, ou
    (b)   on extrait au moyen d'une résine adsorbante à base de polystyrène, on élue la résine adsorbante avec de l'acétone et on sépare la phase d'extrait,

(III)  à partir de la phase d'extrait:
    (a)   on chasse par distillation l'acétone ou l'acétate d'éthyle,
    (b)   on sèche le résidu obtenu (avant ou après avoir chassé le solvant organique par distillation),
    (c)   on soumet le résidu à un partage entre une phase organique polaire et une phase organique non polaire,
    (d)   on concentre la phase organique polaire,
    (e)   on chromatographie la phase organique polaire concentrée, cette chromatographie étant effectuée par exemple sur Sephadex LH-20, et
    (f)   on concentre la phase d'éluant, et

(IV) on chromatographie le mélange de substances actives obtenu sur une colonne garnie d'un gel de silice à inversion de phases au moyen d'un solvant organique polaire additionné d'eau, on sépare par fractionnement la substance active voulue et on l'isole par évaporation du solvant, avant ou après le séchage, et éventuellement par cristallisation fractionnée.

8. Mélange de substances actives qui peut être obtenu par les opérations décrites à la revendication 7, étapes (I) à (III).

9. Micro-organisme, en l'expèce Myxococcus virescens DSM 1888.

10. Micro-organisme, en l'espèce Myxococcus virescens DSM 2177.

HPLC-Elutions-Diagramm der Komponenten mit biologischer Wirkung von Mx-v-48. Material: RP-18, 7 μ; Gradient: A(Methanol:Wasser=85/15), B(Methanol:Wasser = 90/10), unterbrochene Linie: %B (0-100%); Fluß: 2 ml/min; Detektor : 254 nm.
Alle Verbindungen, ausgenommen die bei $R_T$= 1.79 min, sind biologisch aktiv.

Fig. 1

Abbildung  : UV-Spektrum von A und A-1 (MeOH)

Fig. 2

Abbildung : IR-Spektrum der Hauptkomponente A, in CHCl$_3$.

Fig. 3

$^1$H-NMR (400 MHz) von A

CHCl$_3$

Fig. 4

Fig. 5

$^1$H-NMR (400 MHz) von A-1

CHCl$_3$

0 064 547

23

TMS

ppm

Fig. 6: $^{13}$C-Spektrum von A-1

Abbildung   :   IR-Spektrum von   Verbindung B, in CHCl$_3$.

Fig. 7

Fig. 3

$^1$H-NMR (400 MHz) von B

CHCl$_3$

HDO

TMS

x4

x2

10   9   8   7   6   5   4   3   2   1   0

0 064 547

Abbildung : IR-Spektrum von Verbindung C, in CHCl₃, identisch mit dem von Verbindung D.

Fig. 9

Fig. 10

$^1$H-NMR (400 MHz) von C

x2

x2

TMS

CHCl$_3$

$^1$H-NMR (400 MHz) von D

Fig. 11

Abbildung : IR-Spektrum von Verbindung E in CHCl₃.

Fig. 12

¹H-NMR (400 MHz) von E

Fig. 13

FIG.14

FIG.15

FIG.16

FIG.17